# EUROPEAN PATENT APPLICATION

(11) **EP 4 268 708 A1**
(43) Date of publication of application: **01.11.2023**
(21) Application number: 22170475.2
(22) Date of filing: 28.04.2022
(51) Int. Cl.: A61B 5/00, A61B 5/103, B82Y 20/00, G02F 1/017, H01L 51/50

(54) **LIGHTING MODULE**

(71) Applicant: Tridonic GmbH & Co. KG, 6851 Dornbirn (AT)
(72) Inventor: MEITZ, Simon Michael, 6850 Dornbirn (AT); DOBOS, Janos, 6850 Dornbirn (AT)
(74) Representative: Thun, Clemens

(57) **Abstract**

A lighting module (100) for use in medical applications, in particular for providing an illumination suitable for visual inspection to detect changes in vital signs, comprises at least one white light LED (12) and at least one red LED (14), said at least one red LED (14) having a peak wavelength between 620nm and 700nm. The at least one white light LED (12) comprises quantum dots.

## Description

The present invention is directed to a lighting module that can be used in clinical observation areas. More particularly, the present invention is directed to a lighting module that provides light suitable for the visual diagnosis of cyanosis.

The quality of illumination in a medical environment plays an important role in providing the necessary visual conditions for a reliable medical examination of a human patient. In hospitals and medical facilities, there are a variety of visual tasks that require the discrimination of colors to detect changes in vital signs. One of the best-known examples is the diagnosis of cyanosis.

The term "Cyanosis" refers to a pathologic condition that is characterized by a change of body tissue color to a bluish-purple grayish, or purple hue. The discoloration usually affects the skin, mucous membranes, lips and fingernails because of having decreased amounts of oxygen bound to the hemoglobin in the red blood cells of the capillary bed. Cyanosis means that muscles, organs, and other tissues may not be getting the oxygen they need to operate properly. It can be a symptom of a dangerous or life-threatening disorder of the organism or, if it persists for a longer period of time, it can also be a sign of chronic diseases.

Generally, in areas that are primarily designed for medical procedures and/or examination, the lighting system should be designed for the efficient undertaking of the procedure. Because cyanosis is a visual cue of a medical symptom based on observable color, lighting conditions and light quality play a significant part in its visual detection. The detection of a 'bluing' of the skin depends largely on the accurate rendition of both 'normal' skin tones and blue hues, so it is particularly important that the light colour spectrum (and therefore, Colour Rendering Index - CRI) factors are considered when developing a lighting design.

The suitability of a light source for the purpose of visual detection of the presence or onset of cyanosis is usually ranked by an open-ended numerical scale, which is called the Cyanosis observation index (COI) and has been established by the Interior Lighting Standard AS/NZS 1680.2.5:1997. The lower the value of the index, the better suited is the light source for the visual detection of cyanosis, that is, the smaller shift in color appearance that will result under the particular light source when compared with a reference source.

Current lighting solutions providing a sufficient COI either use standard white LEDs combined with red LEDs or a specific combination of RGB LEDs. To enable a feasible observation of cyanosis, the skin illumination has to have a significant part in the red wavelength range of 620 to 700 nm, which is the reason why red LEDs are added to the standard white LEDs. However, the modern requirements of high efficiency and high CRI counteract some of that, meaning that a light source fulfilling all three of these quality features - low COI, high CRI and high efficiency - would be beneficial.

The object underlying the present invention is to provide a new light source, which overcomes this problem.

This problem is solved by a lighting module having the features as defined in independent claim 1. Preferred embodiments of the invention are defined in the dependent claims.

By combining white LEDs utilizing Quantum Dot (QD) technology (allowing to obtain high efficiency and high CRI) with red LEDs having a peak wavelength between 620 and 700 nm (resulting in a good COI), an LED lighting module can be constructed with a total spectrum fulfilling indeed all three quality features mentioned above.

Consequently, according to the present invention, a lighting module is provided for use in medical applications, wherein said lighting module comprises:
- at least one white light LED and
- at least one red LED having a peak wavelength between 620nm and 700nm
wherein the at least one white light LED comprises quantum dots. Preferably, the at least one red LED has a peak wavelength between 620nm and 630 nm or between 650nm and 670nm.

The exact ratio of the number of white light LEDs to the number of red LEDs used depends on the type of the used LEDs. Nevertheless, this ratio preferably is between 5:1 and 14:1, more preferably between 9:1 and 12:1, as it has been shown that the finally emitted light can fulfil all three quality features mentioned before in case the numbers ratio is within this mentioned range.

Further preferably, the ratio of the power consumption of the at least one white light LED to the power consumption of the at least one red LED is between 15:1 and 40:1, more preferably between 26:1 and 35:1, and the ratio of the radiated power of the at least one white light LED to the radiated power the at least one red LED is between 9:1 and 24:1, more preferably between 16:1 and 21:1.

According to another preferred embodiment of the present invention, the ratio of light flux of the at least one white light LED to the light flux of the at least one red LED is between 50:1 and 138:1, in particular between 89:1 and 119:1.

Also according to a preferred embodiment of the present invention, the ratio of radiated power below 620 nm to the radiated power above 620 nm is between 2,3:1 and 2,9:1, preferably between 2,6:1 and 2,8:1.

With regard to the physical structure of the lighting module according to the invention, the at least one white light LED and the at least one red LED are preferably arranged on a common carrier element, wherein the common carrier element can in particular be formed by a printed circuit board (PCB).

In another preferred embodiment of the present invention, the carrier element is provided with cooling structures and/or thermally coupled to a cooling element. This measure ensures that the LEDs of the inventive lighting module can be operated permanently within a predetermined temperature range, thereby ensuring that the spectral composition of the emitted light remains stable.

The quantum dots of the at least one white light LED are preferably used to convert light of a monochromatic blue or violet LED into light of a different wavelength. In particular, the quantum dots convert the light of the monochromatic blue or violet LED into red light, wherein preferably the white light LED additionally comprises a garnet phosphor, e.g., YAG or LuAG or YGdAG, emitting in the greenish-yellow wavelength range.

Preferably, the light module according to the invention comprises a cover element that covers the at least one white light LED and the at least one red LED. This cover serves not only to protect the LEDs, but can also be used to further improve the light emission. In particular, the cover element may be designed to form a diffuser through which the light emitted by the LEDs is mixed. For this purpose, the cover may have light-diffusing structures on at least one of its surfaces, the light-diffusing structures preferably being formed by microprisms.

The present invention also provides a luminaire for medical purposes, in particular for providing an illumination suitable for visual inspection to detect changes in vital signs, wherein the luminaire comprises a lighting module as explained above.

In the following, the inventive concept is discussed in more detail with reference to the accompanying drawings:
- Figure 1: shows a perspective view of a lighting module according to a preferred embodiment of the present invention.
- Figure 2: shows the lighting module of Figure 1 wherein the protective cover has been removed.
- Figure 3: shows a cross-sectional view of the inventive lighting module.
- Figure 4: schematically shows the reflectance of cyanosed blood and oxygenated blood.
- Figure 5: shows the spectral composition of light emitted by the inventive lighting module wherein the highest possible amount of red light is used.
- Figure 6: shows the spectral composition of light emitted by the inventive lighting module wherein the lowest possible amount of red light is used.

Before a preferred embodiment of the present invention is discussed in detail with respect to the drawings, at first the technical idea of using quantum dots for realizing white light LEDs will be briefly explained.

An emission in the green or red color range can be achieved by using respective monochromatic LEDs or a suitable color converting material in combination with one or more blue (or UV or violet) LEDs. Suitable color conversion materials for producing light in the green and/or yellow wavelength range are phosphors having a peak wavelength in the green and/or yellow wavelength range, such as garnet phosphors e.g. LuAG: Ce3+ or YAG: Ce3+(Y3Al5O12: Ce3+) or (Y, Gd)3 Al5O12:Ce3+)), an ortho- silicate e.g. (Ba2SiO4:Eu2+, (Ba,Sr)2SiO4:Eu2+) or β-SiAlON: Eu2+. For emitting light in the red wavelength range, suitable color conversion materials, which have a peak in the red wavelength range, are nitrides ((Sr,Ca)AlSiN3: Eu2+ or CaAlSiN3: Eu2+) or KSF (K2SiF6: Mn4+). Beside red phosphors, Quantum Dots emitting in the red wavelength range could be applied as well.

Quantum Dots (QDs) are semiconductor particles a few nanometers in size, having optical and electronic properties that differ from larger particles due to quantum mechanics. When quantum dots are energized, for example illuminated by UV light, an electron in the quantum dot can be excited to a state of higher energy. In the case of a semiconducting quantum dot, this process corresponds to the transition of an electron from the valence band to the conductance band. The excited electron can drop back into the valence band releasing its energy by the emission of light. The color of that light depends on the energy difference between the conductance band and the valence band, or transition between discretized energy states when band structure is no longer a good definition in QDs.

Quantum dots thus behave similarly to atoms, but their shape, size or the number of electrons in them can be influenced. This allows electronic and optical properties of quantum dots to be tailored. For example, larger quantum dots of 5-6 nm diameter emit longer wavelengths, with colors such as orange or red. In particular, quantum dots emitting in the red wavelength regime are about 6 to 10 nm in size. Smaller quantum dots (for example 2-3 nm) emit shorter wavelengths, yielding colors like blue and green. However, the specific colors vary depending on the exact composition of the quantum dot material/compound. Well-known and applied inorganic QDs are e.g. CdS, CdSe, CdTe, ZnS, ZnSe, InP as core and combined as core-shell nanoparticles with and without additional stabilizing agents. Depending on their particle size, structure and composition they emit in different wavelength ranges from green to red. Perovskite nanodots are also used nowadays in different wavelength ranges. A great advantage of those could be that they are not toxic.

Generally, the following two mechanisms exist for the use of quantum dots in light emitting devices:
- In a first alternative, photoexcitation is used with a traditional primary light source LED (typically blue or ultraviolet, i.e., short wavelength emitting light). The blue LED is then coated with quantum dots that emit in the selected wavelength range e.g. red in response to the excitation of the blue LED. The emission of the e.g. blue LEDs, the selected green/yellow phosphors and the emission of the red QDs results in a white light emission. This process usually provides a warm, yellowish-white light similar to that produced by incandescent lamps and the usage of the quantum dots is comparable to the conventional phosphors mentioned above.
- In "real quantum dot light emitting diodes", the light of a certain wavelength is generated in the device itself by an electro-optical effect of the quantum dots, i.e. the excitation of the quantum dots is not done by light or photons but by electrical energy supplied from outside. The structure of such a QLED is similar to that of OLEDs. Accordingly, a material layer containing quantum dots is sandwiched between layers of electron transport and hole transport materials. An applied electric field causes electrons and holes to move into the quantum dot layer and recombine there, resulting in the emission of a photon.

The use of quantum dot technology therefore makes it possible to provide white-light LEDs with specifically adapted spectral properties. The spectrum of the light finally emitted contains only a small proportion of unwanted wavelengths, so that efficiency can be significantly increased compared to classic solutions for white-light LEDs.

The present invention now makes use of these specific properties of quantum dots to finally provide a light source which can be used in clinical observation areas and fulfills the three requirements mentioned above, i.e., low COI, high CRI and - at the same time - high efficiency.

In particular, to reach a higher efficacy for the white light LEDs, either all phosphors (RGY) or only the red one is replaced by quantum dots of the respective size (and/or composition). Emission of QDs in any wavelength range provides a narrow emission band (it is similar to monochromatic LEDs). The disadvantage of replacing all conventional phosphors, meaning a color conversion only through quantum dots, is the high prize of those quantum dots. Therefore, commercially available QD-white-LEDs (which are available e.g. from Osram) only replace the red phosphor by quantum dots, resulting in a hybrid solution. Nevertheless, also this hybrid solution allows to improve the properties of a light source in particular in cases where it is used for clinical observation, as will be explained in the following.

Therefore, in one of the preferred embodiments the white light emitting LED comprises at least one monochromatic blue or violet LED, a garnet phosphor (YAG or LuAG or YGdAG) emitting in the greenish-yellow wavelength range and red QDs.

Clinical observation areas are areas of a hospital or other medical facility where it may be necessary to assess a patient's condition through visual observation. In particular, there are a variety of visual tasks in hospitals and medical facilities that require the discrimination of colors. These tasks include, for example, visual observations of the following types:
a) Examination of patients' skin coloration in order to detect conditions such as cyanosis and jaundice.
b) General examination of patients for dermatological conditions.
c) The use of color-based diagnostic tests.

The degree of difficulty in color discrimination varies from task to task. The most critical task is the early and positive detection of cyanosis because onset of this condition may occur rapidly and unexpectedly, with life threatening consequences.

Consequently, the color characteristics of the electric light sources used in the areas involved play a significant role in providing the necessary visual conditions for color discrimination tasks. Light sources should have a correlated color temperature (CCT) between 3300 K and 5300 K and a CIE general color rendering index (Ra) > 80. Further, in all areas where it is decided that visual conditions appropriate to the detection of cyanosis are to be provided, light sources used should have a cyanosis observation index (COI) of not greater than 3.

As explained above, the cyanosis observation index (COI) is an open-ended numerical scale ranking the suitability of a light source for the purpose of visual detection of the presence or onset of cyanosis. The lower the value of the index, the better the light source is deemed to be for that purpose.

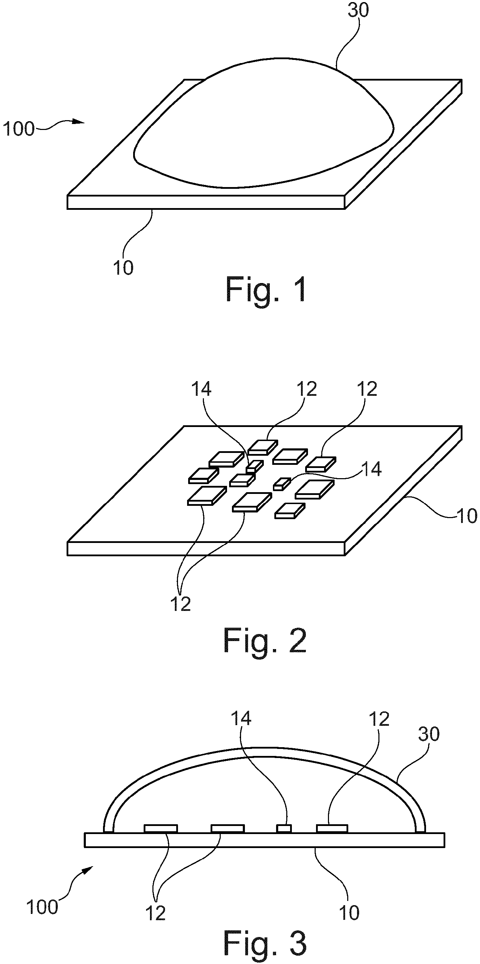

An embodiment of a lighting module according to the present invention is schematically shown in figures 1 to 3.

The inventive lighting module is generally designated 100 and comprises a carrier element 10 supporting several light emitting devices 12, 14 which are collectively covered by a cover 30. The dome-shaped cover 30 not only provides a protection for the light emitting devices 12, 14 but also has the function of a light influencing element that ensures that light is emitted evenly and uniformly in a desired direction. Therefore, the cover 30 preferably has light scattering properties in order to evenly mix the light emitted by the different light emitting elements 12, 14. These scattering properties may be realized by using a material for manufacturing the cover 30 comprising light scattering elements and/or by providing a corresponding light scattering structure on the inner and/or out surface of cover 30. In the later case, the light scattering structure may be formed by microprisms or comparable structures.

Carrier element 10 preferably is formed by a printed circuit board (PCB) which directly carries the light emitting devices 12, 14. In this case, the carrier element comprises contact areas on which the light emitting devices 12, 14 are soldered, as well as associated conductor traces which provide a current supply for the light emitting devices 12, 14. Alternatively, it would also be possible to use light emitting devices 12, 14 which are individually mounted on their own printed circuit boards wherein these elements are then arranged on carrier element 10. In this case, the main function of carrier element 10 is to provide a mechanical support for the electrical components of the lighting module 100, in particular for the plurality of light emitting devices 12, 14.

In the embodiment shown in the figures, carrier element 10 has a square shape. Obviously, it would also be feasible to adapt the dimensions and the shape of the carrier element 10 and thus also of the lighting module 100 depending on the desired form of the resulting light source and/or the total amount of desired light output.

Although not shown in the figures, the support element 30 could also be provided with cooling structures or connected to external cooling elements to dissipate heat generated during operation of the illumination module 100. Since the light emission of semiconductor devices often depends on their temperature, it is important to operate these devices within predefined temperature ranges to ensure that the spectral characteristics of the emitted light meet the desired requirements. It is obvious that this aspect is particularly essential in the field of application of the light module 100 according to the invention, since the spectral stability of the emitted light is of particular importance in such medical lights.

As most important aspect of the present invention, the lighting module 100 comprises two different types of light emitting devices 12, 14. In particular, the lighting module 100 comprises at least one white light LED 12 and at least one red LED 14. Preferably, a plurality of white light LEDs 12 are combined with a plurality of red LEDs 14, the ratio of the numbers of each type of LED being selected, as will be explained in more detail below. The total number of LEDs 12, 14 used depends on the desired total light output. When multiple LEDs 12, 14 of each type are used, the various LEDs 12, 14 are preferably distributed approximately evenly on the support element 10 to ensure that the light is pre-mixed before entering the cover 30.

The red light LEDs 14 preferably emit light with a peak wavelength between 620nm and 700nm. Particularly preferably, the peak wavelength of these LEDs is between 620nm and 630nm or between 650nm and 670nm for the following reason.

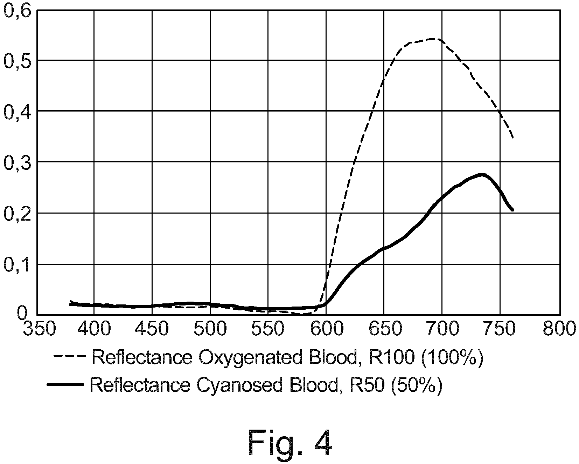

To be able to recognize cyanosis, the light source spectrum has to have a significant part in the range where the reflectance of R50 (i.e., the reflectance of cyanosed blood) and R100 (i.e., the reflectance of oxygenated blood) is different. As can be seen in figure 4, the range for that is between 600nm and 780nm with a maximum difference at about 625nm and in particular at about 660 nm.

One measure of this difference is the Cyanosis Observation Index (COI), which is defined in AS NZS Standard 1680.2.5-2018. For medical lighting under this standard, the following criteria must be met:
- COI <= 3.3
- 3300 K < CCT < 5300 K
- CRI >= 80

By combining white and red standard LEDs, these criteria can be met in principle. However, these solutions do not offer a high CRI (> 90) and high luminous efficacy (> 175 lm/W) at the same time. The reason for this is that with standard white LEDs, efficiency decreases as the CRI increases.

According to the present invention, this disadvantage is overcome by using white LEDs 12 utilizing quantum dot (QD) technology. As explained above, these quantum dots allow to fine tune the fluorescence part of the spectrum in a way that both high CRI and high efficacy can be obtained. When using these quantum dot white LEDs 12 in combination with monochromatic red LEDs 14, the medical lighting criteria can be fulfilled with a high luminous efficacy.

As an additional advantage of the inventive concept, the light source can be tailored to a planned application by changing the relation between quantum dot white LEDs 12 and red red LEDs 14 wherein still the criteria mentioned above are met.

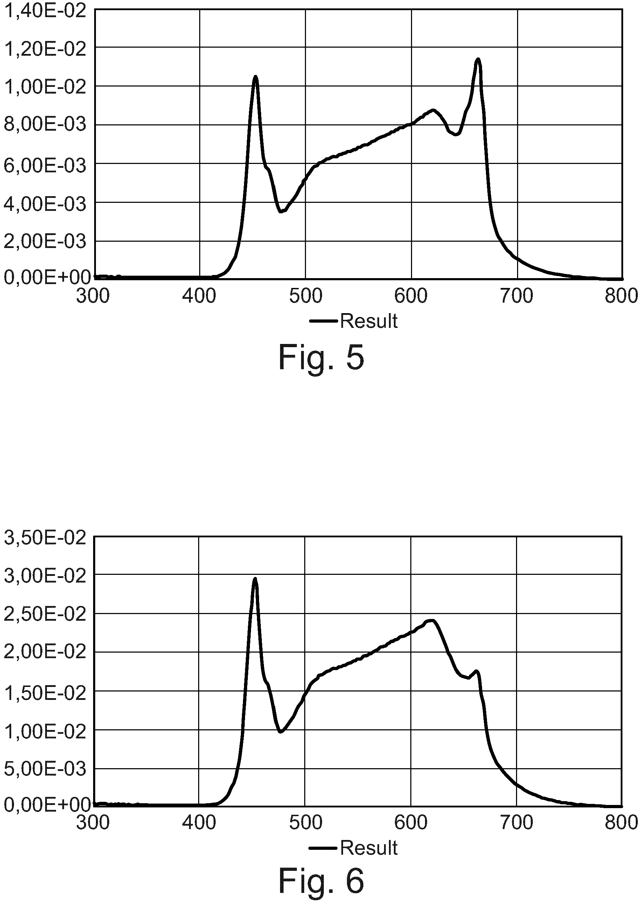

As an example, figure 5 shows the spectrum of an inventive lighting module 100 wherein the highest possible amount of red light is used. Here, the relation of white light LEDs 12 to red LEDs 14 (with a peak wavelength of 660nm) is 5:1; the relation of power consumption is 15:1. In this case, a COI < 1.5 is obtain wherein the efficacy is around 175 lm/W. The resulting color temperature is around 3880K.

In contrast to this first example, figure 6 shows the corresponding spectrum for the case where the lowest amount of red light is used. Now, the relation of white light LEDs 12 to red LEDs 14 is 14:1 and the relation of power consumption is 40:1. The resulting COI in this case is 3,27 - which is still in line with the criteria mentioned above - and the color temperature is around 4050K. The efficacy now is around 180 lm/W due to the higher amount of high efficient quantum dot white LEDs 12.

Based on the above examples, the requirements for the quantum dot white LEDs : red LEDs relation are summarized in the following table:

| **Type of relation** | **Maximum red amount** | **Minimum red amount** |
|---|---|---|
| Number of LEDs | 5:1 | 14:1 |
| Power consumption | 15:1 | 40:1 |
| Radiated power | 9:1 | 24:1 |
| Light flux | 50:1 | 138:1 |
| <= 620 nm : > 620 nm | 2,3:1 | 2,9:1 |
| COI | 1,44 | 3,27 |
| Efficacy | 176 lm / W | 181 lm /W |
| CCT | 3878K | 4046K |
| CRI | 95,83 | 90,32 |

The fifth row describes the relation of radiated power below 620 nm to the radiated power above 620 nm. This is the preferred relation, because it does not depend on a specific embodiment.

These two examples show that it is possible to fine-tune the final light emission of the lighting module by adjusting the ratio of white light to red light, meeting the requirements of AS NZS Standard 1680.2.5-2018, as long as the selected ratios are within the above ranges.

In a preferred embodiment, the relations regarding quantum dot white LEDs : red LEDs are as follows:

| **Type of relation** | **Preferred maximum red amount** | **Preferred minimum red amount** |
|---|---|---|
| Number of LEDs | 9:1 | 12:1 |
| Power consumption | 26:1 | 35:1 |
| Radiated power | 16:1 | 21:1 |
| Light flux | 89:1 | 119:1 |
| <= 620 nm : > 620 nm | 2,6:1 | 2,8:1 |

In case the relationships are selected to be within these narrower ranges, the efficacy of light emission is always higher than 180 lm/W, and the COI is always below 3.0 ensuring an optimized illumination of clinical observation areas.

Accordingly, based on the above considerations and by using white light LEDs comprising quantum dots, it is possible to provide a light source which can be used in clinical observation areas and which fulfils all medical lighting criteria with a high luminous efficacy.

## Claims

1. Lighting module (100) for use in medical applications, in particular for providing an illumination suitable for visual inspection to detect changes in vital signs, wherein said lighting module (100) comprises
- at least one white light LED (12) and
- at least one red LED (14), said at least one red LED (14) having a peak wavelength between 620nm and 700nm,
wherein the at least one white light LED (12) comprises quantum dots.

2. Lighting module according to claim 1,
wherein the at least one red LED (14) has a peak wavelength between 620nm and 630 nm or between 650nm and 670nm.

3. Lighting module according to claim 1 or claim 2,
wherein the ratio of the number of white light LEDs (12) to the number of red LEDs (14) is between 5:1 and 14:1, preferably between 9:1 and 12:1.

4. Lighting module according to one of the proceeding claims,
wherein the ratio of the power consumption of the at least one white light LED (12) to the power consumption of the at least one red LED (14) is between 15:1 and 40:1, preferably between 26:1 and 35:1.

5. Lighting module according to one of the proceeding claims,
wherein the ratio of the radiated power of the at least one white light LED (12) to the radiated power the at least one red LED (14) is between 9:1 and 24:1, preferably between 16:1 and 21:1.

6. Lighting module according to one of the proceeding claims,
wherein the ratio of light flux of the at least one white light LED (12) to the light flux of the at least one red LED (14) is between 50:1 and 138:1, preferably between 89:1 and 119:1.

7. Lighting module according to one of the proceeding claims,
wherein the ratio of radiated power below 620 nm to the radiated power above 620 nm is between 2,3:1 and 2,9:1, preferably between 2,6:1 and 2,8:1.

8. Lighting module according to one of the proceeding claims,
wherein the quantum dots of the at least one white light LED (12) are used to convert light of a monochromatic blue or violet LED into light of a different wavelength.

9. Lighting module according to claim 8,
wherein the quantum dots convert the light of the monochromatic blue or violet LED into red light,
and wherein preferably the white light LED additionally comprises a garnet phosphor, e.g., YAG or LuAG or YGdAG, emitting in the greenish-yellow wavelength range.

10. Lighting module according to one of the proceeding claims,
wherein the at least one white light LED (12) and the at least one red LED (14) are arranged on a common carrier element (10), wherein said common carrier element (10) is preferably formed by a printed circuit board.

11. Lighting module according to claim 10,
wherein the carrier element (10) is provided with cooling structures and/or is thermally coupled to a cooling element.

12. Lighting module according to one of the proceeding claims,
further comprising a cover element (30) covering the at least one white light LED (12) and the at least one red LED (14).

13. Lighting module according to claim 12,
wherein the cover element (30) forms a diffusor.

14. Lighting module according to claim 13,
wherein the cover element (30) has light diffusing structures on at least one of its surfaces, said light diffusing structures preferably being formed by microprisms.

15. Luminaire for medical purposes, comprising a lighting module (100) according to one of the proceeding claims.
